# EUROPEAN PATENT APPLICATION

(11) **EP 3 964 211 A1**
(43) Date of publication of application: **09.03.2022**
(21) Application number: 20194854.4
(22) Date of filing: 07.09.2020
(51) Int. Cl.: A61K 31/4166, A61K 31/455, A61K 31/665, A61K 35/74, A61P 17/10

(54) **A COMPOSITION COMPRISING AN ASCORBYL PHOSPHATE, NIACINAMIDE AND ALLANTOIN FOR TREATING ACNE**

(71) Applicant: DSM IP Assets B.V., 6411 TE Heerlen (NL)
(72) Inventor: SFRISO, Riccardo, 4303 Kaiseraugst (CH); LAURENT, Guillaume Bernard, 4303 Kaiseraugst (CH)
(74) Representative: Berg, Katja

(57) **Abstract**

The present invention relates to a composition comprising an ascorbyl phosphate, niacinamide and allantoin as an anti-microbial agent against *Cutibacterium acnes* (C. *acnes*) as well as the use thereof in a regimen to prevent and/or treat acne comprising the steps of topically administering a composition comprising an ascorbyl phosphate, niacinamide and allantoin to the skin of a subject with acne followed by topically administering a composition comprising one or more non-pathogenic live bacterial C. *acnes* strains.

## Description

The present invention relates to a composition comprising an ascorbyl phosphate, niacinamide and allantoin as an anti-microbial agent against *Cutibacterium acnes* (*C*. *acnes*) as well as the use thereof in a regimen to prevent and/or treat acne comprising the steps of topically administering a composition comprising an ascorbyl phosphate, niacinamide and allantoin to the skin of a subject with acne/acne prone skin followed by topically administering a composition comprising one or more non-pathogenic live bacterial *C. acnes* strains.

Skin is the home for a vast variety of microorganisms, including archaea, bacteria, fungi, viruses and arthropods, which together make up what is known as skin microbiome. The skin microbiome majorly comprises Actinobacteria, Firmicutes, Proteobacteria and Bacteroidetes. The microbiome of epithelia and sebaceous follicles comprises Gram-positive bacteria such as Staphylococcus epidermidis and Cutibacterium acnes (C. acnes hereafter), and fungal species such as Malassezia. As skin commensals, *C. acnes* and *S*. *epidermidis* interact with the host, helping protect healthy skin from colonization by pathogens such as Staphylococcus aureus. An imbalance in the microbiome composition (also called dysbiosis), however, may lead to skin ailments such as acne, eczema, psoriasis, rosacea, and keratosis pilaris.

Acne (common term for Acne vulgaris) is a common inflammatory chronic skin problem, affecting the pilosebaceous units of hair follicles. About 85% of adolescents and young adults are estimated to have been affected by this disease. Acne generally manifests in inflamed papules, pustules or nodules. The inflammation may furthermore be associated with reddening, swelling and pressure pain.

It has now recently been found, that acne is highly correlated with the presence of certain (in the following called pathogenic) *C. acnes* strains, while others, in the following referred to as non-pathogenic *C. acnes* strains do not lead to any adverse skin effects. In this context, WO-2016172196 proposes approach to treat acne which involves the consecutive steps of topically administering a disinfectant or antibiotic to the skin of a subject with acne to significantly reduce the population of *C. acnes* followed by topically administering one or more live, non-pathogenic *C. acnes* strain to said skin (also referred to herein as 'the transplantation of healthy *C. acnes* strains'). This treatment results in the non-pathogenic *C. acnes* strains becoming part of the natural skin microbiome and can thus be used to treat or prevent acne or to maintain skin in a condition where it is free of acne

Suitable disinfectants and antibiotics to kill *C. acnes* for the method as disclosed in WO-2016172196 are salicylic acid, benzoyl peroxide, doxycycline and erythromycin. While salicylic acid and benzoyl peroxide may cause skin irritation, the use of antibiotics contributes to the risk of bacterial resistance. Thus, there is an ongoing need for mild and effective alternatives, which can be used in the preparation of the skin for the above mentioned transplantation of healthy *C. acnes* strains.

Surprisingly, it has now be found that a mixture an ascorbyl phosphate, niacinamide and allantoin synergistically reduces the growth of *C. acnes* and can thus be used for the preparation of the skin for the above mentioned transplantation of healthy *C. acnes* strains. Furthermore said combination can also be used for a conventional treatment of the ailments of acne, which involves an overall reduction of *C. acnes* strains on skin such as the treatment of inflammation triggered by *C. acnes,* red nodules or pustules.

Thus, in one aspect, the invention relates to a composition comprising ascorbyl phosphate, niacinamide and allantoin for use as an antimicrobial agent against *C. acnes.*

In a further embodiment, the invention relates to a method for killing and/or reducing or inhibiting the growth of *C. acnes,* said method comprising contacting *C. acnes* with a combination of ascorbyl phosphate, niacinamide and allantoin.

Due to the antimicrobial activity against *C. acnes* the combination of ascorbyl phosphate, niacinamide and allantoin is further suitable for the treatment of any adverse skin condition associated with (an overpopulation of) *C*. *acnes* by maintaining skin homeostasis and/ or improving the health of the skin microbiome in particular by modulating the skin microbiome of an individual.

The invention also relates to a method of treating the skin and/ or the scalp, said method comprising the steps of topically contacting the skin and/ or scalp with a composition comprising ascorbyl phosphate, niacinamide and allantoin for the treatment, prevention and/or prophylaxis of acne as well as for maintaining skin homeostasis and/or modulate the skin microbiome of an individual (also referred to as microbiome balancing).

In a further embodiment, the present invention relates to the topical use of a composition comprising ascorbyl phosphate, niacinamide and allantoin for the treatment, prevention and/or prophylaxis of acne as well as for maintaining skin homeostasis and/or or modulate the skin microbiome of an individual (also referred to as microbiome balancing).

It is well understood, that all methods and uses according to the present invention can be therapeutic (e.g. for the treatment of acne) as well as merely cosmetic , e.g. to prevent acne and acne rebound effect in acne prone skin, to maintain healthy skin, to maintain skin homeostasis and/or to modulate the skin microbiome.

In all embodiments of the present invention, the compositions according to the present invention comprising an ascorbyl phosphate, niacinamide and allantoin preferably comprise
(a) at least 2 wt. %, preferably 2 to 4 wt. %, most preferably 2.5 to 3.5 wt.-% of an ascorbyl phosphate,
(b) at least 2 wt. %, preferably 2 to 10 wt. %, most preferably 2.5 to 4 wt.-% of niacinamide,
(c) at least 0.25 wt.-%, preferably 0.25 to 1 wt.-%, most preferably 0.3 to 0.75 wt.-%, of allantoin, and
(d) a cosmetically acceptable carrier.

A particularly suitable composition for the purposes of the present invention comprises 3 wt.-% of sodium ascorbyl phosphate, 3 wt.-% of niacinamide and 0.5 wt.-% of allantoin.

If nothing else is stated in this specification any given parts and percentages are per weight and based on the total weight of the composition.

The term allantoin as used herein refers to a compound of formula (1)

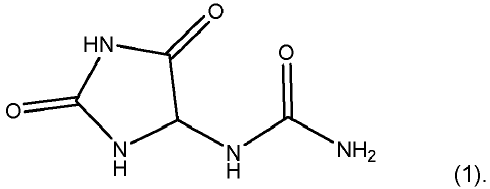

It is also known as 2,5-dioxo-4-imidazolidinyl urea, glyoxyldiureide respectively 5-ureido-hydantoine [CAS 97-59-6].

The term "ascorbyl phosphate" as used herein denotes metal salts of mono- and polyphosphoric acid esters of ascorbic acid wherein the phosphorylated hydroxy group of the ascorbic acid molecule features one or more phosphoric acid (phosphate) units, and metal cations, e.g. sodium and/or magnesium or calcium ions, are also present. The term "poly" generally denotes 2 - 10, preferably 2 - 4, phosphate units. The ascorbyl phosphates may also be referred to in general as "ascorbyl (poly)phosphates" to embrace both mono- and polyphosphates. Typical ascorbyl phosphates for use in the present invention are L-ascorbic acid phosphate ester salts such as sodium ascorbyl phosphate, potassium ascorbyl phosphate, magnesium ascorbyl phosphate, calcium ascorbyl phosphate and sodium magnesium L-ascorbyl-2-monophosphate. The ascorbyl phosphates are essentially present in the form of a hydrate or a dihydrate. Commercially available ascorbyl phosphates comprise trisodium L-ascorbyl-2-monophosphate which is available as STAY-C^{®}50 from DSM Nutritional Products AG, (4303 Kaiseraugst, Switzerland) and magnesium L-ascorbyl phosphate (available from Showa Denko) and sodium magnesium L-ascorbyl-2-monophosphate. Preferred ascorbyl phosphates for all the purposes of the present invention are sodium or sodium magnesium or sodium calcium ascorbyl phosphate as well as mixtures thereof. Most preferred in all embodiments of the present invention is the use of trisodium L-ascorbyl-2-monophosphate dihydrate.

Niacinamide [CAS-Nr. 98-92-0] is one of the water-soluble B-complex vitamins which is e.g. available as Niacinamide PC or Niacinamide from DSM Nutritional Products AG, (4303 Kaiseraugst, Switzerland). Niacinamide is also referred to as nicotinamide or pyridine-3-carboxamide and is the amide of niacin (vitamin B₃).

All compositions according to the present invention are preferably intended for topical use, and even more preferably cosmetic compositions.

The term 'cosmetic composition' as used herein refers to compositions, which are used to treat, care for or improve the appearance of the skin and/or the scalp. Particular advantageous cosmetic compositions according to the present invention are skin care preparations.

The term 'cosmetically acceptable carrier' (also referred to herein as carrier) refers to all vehicles/ carriers conventionally used in cosmetic compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art.

The exact amount of carrier will depend upon the actual level of the actives (a) to (c) and any other optional ingredients that one of ordinary skill in the art would classify as distinct from the carrier (e.g., other active ingredients).

In an advantageous embodiment, the cosmetic compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

Particularly suitable topical compositions according to the invention are leave-on or rinse off products, and include any product applied to the human body. The composition can be in the form of a liquid, lotion, cream, foam, scrub, gel, soap bar or toner, or applied with an implement or via a face mask, pad or patch. Non-limiting examples of such compositions include leave-on skin lotions and creams, shampoos, conditioners, shower gels, face wash's, body wash's, toilet bars, antiperspirants, deodorants, depilatories, lipsticks, foundations, mascara, sunless tanners and sunscreen lotions.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention can be in a wide variety of forms. Non limiting examples include simple solutions (e.g. aqueous, organic solvent, or oil based), emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. of oil-in-water-in oil (O/W/O) or water-in-oil-in-water (W/O/W) type) or pickering emulsions), as well as solid forms (e.g. hydrogels, alcoholic gels, lipogels, sticks, flowable solids, or amorphous materials).

These product forms may be used for a number of applications, including, but not limited to, gels, creams, ointments, lotions, serums, powder, aerosol sprays or two component dispensing systems.

If the composition is an emulsion, such as in particular an O/W-, W/O-, Si/W-, W/Si-, O/W/O-, W/O/W- or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

In one embodiment, the compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are polyalkylene glycol ethers, sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C 10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate as well as polyalkylene glycol ethers such as in particular polyethylene stearyl ethers such as Steareth-2 and Steareth 21.

A particular suitable class of O/W emulsifier to be used in the compositions according to the invention are polyalkyleneglycolethers. Particularly preferred O/W emulsifier in all emodiments of the present invention are the stearyl ethers of polyethyleneglycol, such as most preferably Steareth-2 (Polyoxyethylen (2) stearylether) or Steareth-21 (Polyoxyethylen (21) stearylether) as well as mixtures thereof. Such polyalkyleneglycolethers emulsifiers are e.g. commercially available under the Brij tradename at Croda.

In another particular advantageous embodiment, the cosmetic compositions according to the present invention are clear serums. Such clear serums preferably consist essentially of allantoin, niacinamide, an ascorbylphosphate, water, a thickener and a preservative. It is furthermore particularly preferred that the thickener is xanthan gum.

The cosmetic compositions according to the present invention advantageously comprise a preservative. Particular suitable preservatives in all embodiments of the present invention are phenoxyethanol and ethylhexylglycerin as well as mixtures thereof. When present, the preservative is preferably used in an amount of 0.1 to 2 wt.-%, more preferably in an amount of 0.5 to 1.5 wt.-%, based on the total weight of the composition.

The compositions according to the invention in general have a pH in the range of 3 to 10, preferably a pH in the range of 4 to 8 and most preferably a pH in the range of 5 to 8. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

Preferred topical compositions according to the present invention are rinse-off preparations, which are intended/required to be removed from the body by washing with solvent, preferably water, after the application of said composition.

Preferred rinse-off compositions in all embodiments according to the present invention are detergent rinse-off compositions, which are used for cleansing the skin. Such detergent rinse-off composition can either be solid such as e.g. in the form of a soap bar or in a liquid form such as e.g. a shower gel, a wash gel, a hair shampoo, a body shampoo, a foam bath, a shower bath or a shaving preparation or in the form of a foam such as e.g. a shaving foam. It is well understood by a person skilled in the art, that in case of detergent rinse-off compositions, the cosmetically acceptable carrier further comprises at least one surfactant and/or at least one soap.

Particularly preferred detergent rinse-off compositions according to the present invention are liquid detergent rinse of compositions.

Preferably, in all embodiments of the present invention, the detergent rinse-off compositions according to the present invention consist essentially of a saccharide isomerate, water, at least one, preferably several soaps and/ or surfactants, moisturizers, chelating agents, basifying or acidifying agents, actives, solubilizers, pearlescent or opacifying agents, thickening agents, humectants, and additives which enhance their appearance, feel and fragrance, such as colorants, fragrances, preservatives, and the like.

The water content of the detergent rinse-off compositions according to the present invention is preferably selected in the range from 40 to 70 wt.-%, more preferably in the range from 45 to 65 wt.-%, most preferably in the range from 50 to 60 wt.-%.

The term "soap" is used herein in its popular sense, i.e., it refers to the alkali metal or alkanol ammonium salts of aliphatic, alkane- or alkene monocarboxylic acids as well as mixtures thereof. Sodium, potassium, magnesium, mono-, di- and tri-ethanol ammonium cations, or combinations thereof, are particularly suitable for the purposes of this invention, preferably, however, sodium or potassium soaps are used.

Particularly preferred soaps for the purpose of the present invention are the well-known alkali metal salts such as in particular the sodium and/ or potassium salts of natural or synthetic aliphatic (alkanoic or alkenoic) acids having from about 8 to 22 carbon atoms, preferably from about 8 to about 20 carbon atoms, most preferably from about 10 to about 18 carbon atoms. Even more preferred are the salts of the respective saturated (alkanoic acid) acids.

It is furthermore preferred, that the soaps used in the detergent composition according to the present invention comprise at least 85% of fatty acids having from 12 to 18 carbon atoms.

Particularly preferred soaps to be used in the detergent compositions of the present invention are the sodium and/ or potassium salts of stearic acid, lauric acid, myristic acid, oleic acid and palmitic acid.

It is well understood that the soap can be used as such, or can be formed 'in situ' in the detergent rinse-off composition by adding the respective acid and the respective base to the composition.

The amount of the at least one soap in the detergent rinse-off compositions according to the present invention may easily be selected by a person skilled in the art, such as in the range from 3 to 95 wt.-%. It is well understood by a person skilled in the art, that the amount is strongly dependent on the type of detergent rinse-off composition, i.e. if the detergent rinse-off composition is a soap bar the concentration is selected in the range of 50-95 wt.-%, while if the composition is a liquid (aqueous) rinse-off composition the concentration is selected in the range of 15 to 50 wt.-%, preferably in the range from 20 to 40 wt.-%, most preferably in the range from 25 to 35 wt.-%, based on the total weight of the detergent rinse-off composition.

Particularly preferred soaps to be used in detergent the rinse-off compositions according to the present invention are the sodium and/or potassium salts of lauric acid, stearic acid and myristic acid as well as mixtures thereof.

Particularly suitable surfactants to be used in the detergent rinse-off compositions according to the present invention, such as in particular in the liquid detergent rinse-off compositions are anionic, cationic, non-ionic and/or amphoteric surfactants to form a surfactant mixture.

Suitable anionic surfactants to be included into the detergent rinse-off compositions according to the invention include, but are not limited to aliphatic sulphate, aliphatic sulfonate (e. g., C8 to C22 sulfonate or disulfonate), aromatic sulfonate (e. g., alkyl benzene sulfonate), alkyl sulfosuccinates, alkyl and acyl taurates, alkyl and acyl sarcosinates, sulfoacetates, alkyl phosphates, carboxylate and isethionates as well as mixtures thereof.

Particularly suitable anionic surfactants to be used for the purpose of the present invention are alkyl sulfates such as preferably sodium, triethanolamine or ammonium lauryl sulfates; alkyl ether sulfates (or Alkyl PEG-n sulfates) such as preferably sodium or ammonium lauryl ether sulfate, laureth sulfate, sodium C2-15 pareth sulfate; alkyl amido ether sulfates; alkylaryl polyether sulfates; monoglycerides sulfates; acyl isethionate salts such as preferably sodium acylisethionate, sodium cocoyl isethionate; alkylaryl sulfonates salts such as preferably sodium alkylbenzene sulfonate and/ or sodium dodecylbenzene sulfonate; alkyl sulfonates salts such as preferably sodium alkenyl sulfonate (sodium C12-14 olefin sulfonate), sodium alkylglyceride sulfonate (sodium cocomonoglyceride olefin sulfonate), sodium alkylether sulfonate (sodium C12-15 pareth-15 sulfonate) and/ or sodium lauryl sulfoacetate; (di)sodium sulfosuccinates such as preferably sodium dialkyl sulfosuccinate (dioctyl sodium sulfosuccinate), disodium alkyl PEG-n sulfosuccinate, disodium alkylamido PEG-n sulfosuccinate (disodium oleamido MEA-sulfosuccinate), disodium alkylsulfosuccinate; alkyl phosphates (mono-esters) such as preferably TEA monolauryl phosphate; PEG-n alkyl phosphates such as preferably DEA oleth-10 phosphate; di PEG-n alkyl phosphates (di-esters) such as preferably dilaureth-4 phosphate; phospholipids (tri-esters) such as preferably lecithin; carboxylic acids ester, such as preferably mono-ester of di- or tri- carboxylic acids such as lactylates (sodium acyllactylate, calcium stearoyl lactylate), laureth-6 citrate, dinonoxynol-9 citrate; ether carboxylic acids such as preferably sodium PEG-n alkyl carboxylates, sodium trideceth-13 carboxylate, nonoynol-8 carboxylic acid, alkyl C6-C24 ether carboxylates polyoxyalkylenated; acyl glutamates such as preferably di-TEA palmitoyl aspartate and sodium hydrogenated tallow glutamate; Acyl peptides with various amino acids side groups such as preferably palmitoyl hydrolysed milk protein, sodium cocoyl hydrolysed soy protein, TEA-cocoyl hydrolysed collagen or other acyl hydrolysed protein salts; sarcosinates or acyl sarcosides such as preferably myristoyl sarcosine, TEA-lauroyl sarcosinate; as well as taurates and sodium methyl acyltaurates such as preferably sodium lauroyl taurate, sodium methyl cocoyl taurate.

Particularly suitable non-ionic surfactants to be used for the purpose of the present invention encompass ethers comprising aliphatic (C6-C18) primary or secondary linear or branched chain acids, alcohols or phenols which possess no functional grouping other than the terminal OH group of the Polyoxyethylenated (POE) chain as well as ethoxylated alcohols and propoxylated POE ethers such as preferably PEG ethers, PPG ethers, propylene glycol alkyl POE-n ethers; alkyl polyglucosides of the general formula CnH2n+1O(C6H10O5)xH, with x being 1 to 4 such as preferably decyl glucoside, and lauryl glucoside; alkanolamides auch as preferably N-acyl derivatives of monoethanolamine (MEA) and diethanolamine (DEA), ethoxylated or not; such as preferably PEG-n acylamides, coco mono- or di-ethanolamide, palmamide MEA, Acylamide DEA; esters such as preferably ethoxylated fatty acids; mono- and di-esters of fatty acids with ethylene oxide or polyethylene glycol, PEG-n acylate and diacylate such as PEG-8 laurate, PEG-8 dilaurate, PEG-100 stearate, PEG-150 distearate, ethoxylated glycerides such as preferably PEG-n glyceryl acylate, PEG-4 castor oil, PEG-120 glyceryl stearate, triolein PEG-6 esters, glycol esters and derivatives, mono-esters of either ethylene or propylene glycol such as preferably glycol acylate or propylene glycol acylate, monoglycerides such as glyceryl myristate or stearate, glyceryl palmitate lactate, polyglyceryl esters such as polyglyceryl-n acylate or polyglyceryl-n alkyl ether, sorbitan/sorbitol esters such as preferably acetylated sorbitan ethoxylated or not, polysorbate-n, sorbitan sequiisostearate, alkyl carbohydrates esters or sucrose esters resulting from transesterification of sucrose with fatty acid methyl esters or triglycerides such as preferably alkylpolysaccharides; as well as amine oxides such as preferably cocoamidopropyl amine oxide and lauramine oxide. A particularly preferred group of non-ionic surfactant to be used in the rinse-off compositions according to the present invention are the alkyl polyglucosides such as lauryl glucoside, PEG-n acylate and diacylates such as PEG 100 stearate and glyceryl stearate.

Particularly suitable zwitterionic and amphoteric surfactants according to the present invention encompass secondary or tertiary aliphatic amine derivatives with an aliphatic chain, linear or branched, containing at least 8 to 22 carbon atoms and one anionic group selected from the group of carboxylate, sulfonate, sulfate, phosphate or phosphonate; acyl/dialkyl ethylenediamines such as preferably acylamphoacetate, disodium acylamphodipropionate, sodium acylamphohydroxypropylsulfonate, disodium acylamphodiacetate, sodium acylamphopropionate and wherein the acyl group represents either an alkyl or alkenyl group which can be mon- or polyunsaturated and contains from 5 to 29 carbon atoms; N-alkyl amino acids or imino diacids such as preferably aminopropyl alkylglutamide, alkylaminopropionic acid, sodium alkylimino propionate, alkyl glycinates and carboxyglycinates, sodium cocoglycinates; sas well as betaines such as preferably alkyl (C8-C20) betaines, alkyl amidopropyl betaines (cocamidopropyl betaines), alkyl (C8-C20) amidoalkyl (C1-C6) betaines, alkyl sulphobetaines and alkyl (C8-C20) amidoalkyl (C1-C6) sulphobetaines.

Particularly suitable cationic surfactants according to the present invention encompass alkylamines such as preferably dimethyl alkylamine (dimethyl lauramine), dihydroxyethyl alkylamine dioleate, acylamidopropyldimethylamine lactate (cocamidopropyl dimethylamine lactate); alkyl imidazolines such as preferably alkyl hydroxyethyl imidazoline, Ethylhydroxymethyl oleyl oxazoline, alkyl aminoethyl imidazoline; ethoxylated alkylamines such as preferably PEG-n alkylamines, PEG-n Alkylaminopropylamine, poloxamine; quaternary compounds such as preferably tetraalkylammonium salts; alkyl trimonium chloride, PEG-n alkylmonium chloride, dialkyldimonium chloride (hydroxyethyl cetyldimonium chloride), alkylamidopropyl alkyldimonium tosylate (Cocamidopropyl ethyldimonium ethosulfate), PEG-n Acylmethyldiethonium methosulfate, dialkyl hydroxypropylmonium methosulfate, and alkyldimonium hydroxypropyl protein hydrolysate (Cocodimonium hydroxypropyl hydrolysed hair keratin).

Particularly preferred surfactants to be used in the rinse-off compositions according to the present invention are selected from the group glucosides such as e.g. lauroyl glucosides, arachidyl glucoside, caprylyl/capryl glucoside and coco-glucoside, PEG-n acylate and diacylate such as e.g. PEG-8 laurate, PEG-8 dilaurate, PEG-100 Stearate, monoglycerides such as glyceryl myristate or stearate as well as mixtures thereof.

The amount of the at least surfactant in the detergent rinse-off compositions according to the present invention is preferably selected in the range of 1 to 30 wt.-%, preferably 2.5 to 10 wt.-%, most preferably 5 to 15 wt.-%, based on the total weight of the detergent rinse-off composition. Preferably, however, the amount of the surfactant in the rinse-off composition is selected in the range from 15 to 50 wt.-%, preferably in the range from 20 to 40 wt.-%, most preferably in the range from 25 to 35 wt.-%, based on the total weight of the detergent rinse-off composition.

In a particular preferred embodiment, the detergent rinse-off composition according to the present invention comprises a mixture of at least one, preferably several soaps and at least one surfactant with all the definitions and preferences as given herein. Even more preferably the total amount of the mixture of soap(s) and surfactant(s) is selected in the range of 15 to 50 wt.-%, preferably in the range from 20 to 40 wt.-%, most preferably in the range from 25 to 35 wt.-%, based on the total weight of the detergent rinse-off composition. It is then even more preferred that the soap constitutes greater than 60 wt.-%, more preferably greater than 65 wt.-%, most preferably greater than 70 wt. % of said mixture.

Suitable chelating agents to be incorporated into the detergent rinse-off compositions according to the present invention encompass those which are capable of protecting and preserving the compositions of this invention. Preferably, the chelating agent is ethylenediamine tetraacetic acid ("EDTA"), and more preferably is tetrasodium EDTA, available commercially from Dow Chemical Company of Midland, Michigan under the tradename, "Versene 100XL" or even disodium EDTA, commercially available from BASF under tradename "EDETA BD".

Others suitable chelating agents include Phytic acid and its sodium salts, gluconic acid and its sodium salts and etidronic acid and its sodium salts, phosphoric acid and its sodium salts, gluconic acid and its sodium salts, oxalic acid, citric acid, lauryl alcohol diphosphonic acid, tetrasodium glutamate diacetate, trisodium dicarboxymethyl alaninate, trisodium ethylenediamine disuccinate.

The amount of the chelating agent is preferably selected in the range from 0.01 to 1 wt.-%, preferably in the range from 0.1 to 0.75 wt.-%, most preferably in the range from 0.25 to 0.75 wt.-%, based on the total weight of the detergent rinse-off composition.

The acidifying agents can be, for example, mineral or organic acids, for instance hydrochloric acid, orthophosphoric acid, carboxylic acids, for instance tartaric acid, citric acid, lactic acid, or sulphonic acids.

Among the basifying agents which may be mentioned, for example, are the alkaline or earth alkaline salts such as sodium or potassium hydroxide, alkali metal carbonates, as well as alkanolamines such as monoethanolamine, diethanolamine and triethanolamine

The basifying or acidifying agent is used to adjust the pH of the rinse-off compositions. In a preferred embodiment, the pH of the detergent rinse-off composition according to the present invention is adjusted with the basifying or acidifying agent to be in the range from about 4.5 to about 10.5, and more preferably from about 5.0 to about 10.0.

Furthermore, the amount of the basifying or acidifying agent in the detergent rinse-off composition according to the present invention is preferably at least 0.0001 wt.-%, such as e.g. in the range from 0.01 to 6 wt.-%, in particular in the range from 3 to 5 wt. %.

The detergent compositions of the present invention may also include one or more optional ingredients such as a pearlescent or opacifying agent, a thickening agent, humectants, and additives which enhance their appearance, feel and fragrance, such as colorants, fragrances, preservatives, and the like.

Commercially available pearlescent or opacifying agents which are capable of suspending water insoluble additives and/or which tend to indicate to consumers that the resultant product is a detergent composition are suitable for use in this invention. The pearlescent or opacifying agent may be present in an amount, based upon the total weight of the composition, of from about 1 wt.-% to about 10 wt.-%, preferably from about 1.5 wt.-% to about 7 wt.-%, and more preferably, from about 2 wt.-% to about 5 wt.-%.

Examples of suitable pearlescent or opacifying agents include, but are not limited to mono or diesters of (a) fatty acids having from about 16 to about 22 carbon atoms and (b) either ethylene or propylene glycol mono or diesters of (a) fatty acids having from about 16 to about 22 carbon atoms, (b) a polyalkylene glycol of the formula: HO-(JO)a-H, wherein J is an alkylene group having from about 2 to about 3 carbon atoms and a is 2 or 3; fatty alcohols containing from about 16 to about 22 carbon atoms; fatty esters of the formula: KCOOCH2L, wherein K and L independently contain from about 15 to about 21 carbon atoms; inorganic solids insoluble in the detergent composition, and mixtures thereof.

The pearlescent or opacifying agent may be introduced to the detergent composition as a pre-formed, stabilized aqueous dispersion, such as that commercially available from Henkel Corporation of Hoboken, New Jersey under the tradename, "Euperlan PK-3000." This material is a combination of glycol distearate (the diester of ethylene glycol and stearic acid), Laureth-4 (CH3(CH2)10CH2(OCH2CH2)4OH) and cocamidopropyl betaine and preferably is in a weight percent ratio of from about 25 to about 30: about 3 to about 15: about 20 to about 25, respectively.

Commercially available thickening agents, which are capable of imparting the appropriate viscosity to the detergent rinse-off compositions are suitable for use in this invention. If used, the thickener should be present in the compositions in an amount sufficient to raise the Brookfield viscosity of the composition to a value of between about 500 to about 10,000 centipoise. Examples of suitable thickening agents nonexclusively include: mono or diesters of 1) polyethylene glycol of formula: HO-(CH2CH2O)zH, wherein z is an integer from about 3 to about 200; and 2) fatty acids containing from about 16 to about 22 carbon atoms; fatty acid esters of ethoxylated polyols; ethoxylated derivatives of mono and diesters of fatty acids and glycerin; hydroxyalkyl cellulose; alkyl cellulose; hydroxyalkyl alkyl cellulose; and mixtures thereof. Preferred thickeners include polyethylene glycol ester, and more preferably PEG-150 distearate which is available from the Stepan Company of Northfield, Illinois or from Comiel, S.p.A. of Bologna, Italy under the tradename, "PEG 6000 DS".

The amount of thickener(s) in the detergent rinse-off composition is preferably selected in the range from 0 to 7 wt.-%, preferably from 1 to 5 wt.-%, most preferably from 2 to 4 wt.-%, based on the total weight of the detergent rinse-off composition.

Commercially available humectants, which are capable of providing moisturization and conditioning properties to the detergent composition, are suitable for use in the present invention. Examples of suitable humectants nonexclusively include: 1) water soluble liquid polyols selected from the group comprising glycerol, propylene glycol, 1,3 propanediol, hexylene glycol, butylene glycol, dipropylene glycol, and mixtures thereof; 2) polyalkylene glycol of the formula: HO-(R"O)b-H, wherein R" is an alkylene group having from about 2 to about 3 carbon atoms and b is an integer of from about 2 to about 10; 3) polyethylene glycol ether of methyl glucose of formula CH3-C6H10O5-(OCH2CH2)c-OH, wherein c is an integer from about 5 to about 25; 4) urea; and 5) mixtures thereof, with glycerol or PEG-32 being the preferred humectant.

Preferably, in all embodiments, the detergent rinse-off composition comprise at least one humectant. The amount of the at least on humectant, if present in the detergent composition is preferably selected in the range from 0 to 90 wt.-%, preferably from 5 to 40 wt.-%, most preferably from 15 to 30 wt.-%, based on the total weight of the detergent rinse-off composition. Further suitable ranges encompass ranges from 1 to 10 wt.-%, from 2 to 8 wt.-% and from 2.5 to 5 wt.-%, based on the total weight of the detergent rinse-off composition.

Suitable preservatives to be included into the detergent rinse-off compositions according to the present invention include Quaternium-15, available commercially as "Dowicil 200" from the Dow Chemical Corporation of Midland, Michigan, and are present in the composition in an amount ranging from about 0 to 5.0 wt.-%, preferably from about 0.05 to 2 wt.-%, most preferably from about 0.1 to 1.5 wt.-%, based on the total weight of the detergent composition.

The detergent composition of the present invention may be used on the body in conjunction with any personal cleansing implement known in the art such as a washcloth, a mesh or apertured film, pouf, sponge, brush and the like. The composition may be marketed together with one or more of such implements in a kit.

The compositions of the present invention may furthermore be "substantially free" of oils or silicones. As used herein, "substantially free" shall mean that the detergent rinse-off composition contains less than about 1 wt.-%, for example, less than about 0.5 wt.-% or less than about 0.2 wt.-% oils and/or silicones, based on the total weight of the detergent composition.

In another preferred embodiment, the detergent rinse-off compositions according to the present invention are (solid) soap bars.

Most preferred detergent rinse-off compositions according to the present invention consist essentially of a saccharide isomerate, water, at least one, preferably several soaps and/or surfactants and optionally moisturizer(s), chelating agent(s) and a basifying or acidifying agent with all the definitions and preferences as given herein.
Finally, the invention also relates to the use of a composition according to the present invention for the pre-treatment of skin to prepare said skin for the topical administration of a bacteria containing formulation comprising at least one non-pathogenic C. acnes strain and thus for the transplantation of healthy *C. acnes* strains to said skin. Said use can be therapeutically, e.g. for the treatment of acnes, as well as merely cosmetic, e.g. to prevent acne, to maintain healthy skin and/or to modulate the skin microbiome.

In this context, the invention also relates to a regimen to prevent and/or treat acne, said regimen comprising the steps of topically administering a composition comprising an ascorbyl phosphate, niacinamide and allantoin with all the preferences and definitions as given herein to the skin of a subject with acne followed by topically administering a bacteria containing formulation comprising one or more non-pathogenic live bacterial C. acnes strains.

The present invention also relates to the use of a composition comprising one or more non-pathogenic live bacterial strains for treatment of acne or the prevention of acne rebound effect in acne prone skin, wherein the composition is topically administered to the skin of a subject with acne following administration of a composition comprising an ascorbyl phosphate, niacinamide and allantoin with all the preferences and definitions as given herein to the skin of the subject.

In the context of the present invention the term 'non-pathogenic *C. acnes* strains refers to strains which exhibit a slow or negligible conversion or degradation of cis-9, cis-12 linoleic acid in media, such as in particular the *C. acnes* strains D1, A5, C3, H1 (6609), H2, H3, K1, K2, K4, K6, K8, K9, L1 and F4 as outlined and disclosed in WO-2016172196.

Preferably, in all embodiments of the present invention, the one or more live bacterial strains are *C. acnes* bacterial strains selected from the group consisting of: *C. acnes* 6609 (H1), C1, C3, D1, A5, H1, H2, H3, K1, K2, K4, K6, K8, K9, L1 and F4 bacterial strains.

Even more preferably, in all embodiments of the present invention the bacteria containing formulation comprises one or two *C. acne* strains, such as most preferably the *C. acnes* strain C3 and/ or K8. Most preferred is the use of both of said strains (i.e. C3 and K8) in approximately equal proportions. Said bacterial containing formulation may further comprise a peptone.

It is also preferred to combine said strains (i.e. C3 and/or K8) with the *C. acnes* strains A5 and/or F4.

Preferably, the bacteria of the *C. acnes* strains are incorporated into the bacteria containing formulation in the form a lyophilizate. Said lyophilizate can be prepared according to standard methods in the art.

Most preferably, said bacteria containing formulation is a dual vial cosmetic product as disclosed in WO-2019238968 which is incorporated herein by reference.

The following examples are provided to further illustrate the compositions and effects of the present invention. These examples are illustrative only and are not intended to limit the scope of the invention in any way.

### Example

### Cutibacterium acnes Time-Kill assay:

Sodium ascorbyl phosphate, niacinamide, allantoin as well as a combination thereof were screened for their potential anti-microbial activities against *C. acnes* (ATCC reference 11827). The action of ingredients and combinations thereof is assessed through a miniaturized method performed in liquid media in 96 deepwell plates format. The inhibition of the growth of *C. acnes* is compared to the same strain growth in corresponding optimal media.

### Sample preparation and method:

The different ingredients are mixed in growth medium (final volume 2mL) in deepwell plates (n=3).
Analysis controls are prepared and used identically to samples :
- A growth control of *Cutibacterium acnes* in the corresponding culture medium;
- A growth control of *Cutibacterium acnes* with ethanol at 2,5 % (v/v) directly in the culture medium;
- A growth control of microorganisms with olive oil at 5 % (v/v) directly in the culture medium;
- A control consisting of Salicylic acid at 0.5% directly in the culture medium
Then, samples are contaminated with C. acnes, inoculum at final concentration of 3,7 x105 cfu/mL, and incubated at 37°C ± 2,5°C during 24h. At each defined timepoint (i.e. 30min, 2h, 4h, 16h and 24h), a pick-up of the contaminated samples is performed (50 µL) after gentle agitation.

To perform the counting, aliquots of the contaminated samples are serially diluted (decimal dilutions) in culture medium in 96-well plates with triphenyltetrazolium and incubated at 37°C ± 2,5°C during 48 h. After 48 h at the optimal growing conditions, the last dilution showing a pink color (triphenyltetrazolium) or turbidity indicates the contamination rate and allow the determination of the corresponding microbial population.

### Results:

The results of the Time-Kill study are presented in the table below and were compared to the optimal culture medium, the phenonip 0,5% and salicylic acid 0.5%. Data are shown as log-step reduction compared to initial log/cfu of 5.6.

| **Ingredient** | **Concentration (wt.-%)** | **Log-step reduction (2h)** |
|---|---|---|
| Sodium ascorbyl phosphate | 3 | 1.7 |
| Niacinamide | 3 | 0.7 |
| Allantoin | 0.5 | 0.6 |
| Sodium ascorbyl phosphate - Niacinamide - Allantoin | 3-3-0.5 | **3.6** |
| Salicylic acid | 0.5 | 3 |
| Culture medium | - | 0 |

The data reported in the above table shows that the combination of sodium ascorbyl phosphate, niacinamide and allantoin provided a synergistic 3.6 log-step reduction of *C. acnes* in the first 2.5 hours which even outperformed salicylic acid (3 log-step reduction).

## Claims

1. A composition comprising an ascorbyl phosphate, niacinamide and allantoin as an anti-microbial agent against *Cutibacterium acnes.*

2. The composition according to claim 1, wherein the ascorbyl phosphate is selected from the group consisting of trisodium L-ascorbyl-2-monophosphate, magnesium L-ascorbyl phosphate and sodium magnesium L-ascorbyl-2-monophosphate as well as mixtures thereof.

3. The composition according to claim 1, wherein the ascorbyl phosphate is trisodium L-ascorbyl-2-monophosphate.

4. The composition according to anyone of the preceding claims, wherein the composition is a topical composition.

5. The composition according to anyone of the preceding claims for maintaining skin homeostasis and/or modulate the skin microbiome of an individual.

6. The composition according to any one of the preceding claims for the treatment of acne.

7. The composition according to anyone of the preceding claims, comprising
(a) at least 2 wt. %, preferably 2 to 4 wt. %, most preferably 2.5 to 3.5 wt.-% of the ascorbyl phosphate,
(b) at least 2 wt. %, preferably 2 to 10 wt. %, most preferably 2.5 to 4 wt.-% of niacinamide,
(c) at least 0.25 wt.-%, preferably 0.25 to 1 wt.-%, most preferably 0.3 to 0.75 wt.-%, of allantoin, and
(d) a cosmetically acceptable carrier.

8. The composition according to claim 7, wherein the carrier consists of at least 40 wt.-%, more preferably of at least 50 wt.-%, most preferably at least 55 wt.-% of water, such as in particular of about 55 to about 90 wt.-% of water, based on the total weight of the composition.

9. The composition according to anyone of the preceding claims, wherein the composition is a leave-on or a rinse-off composition.

10. The composition according to anyone of the preceding claims for the pre-treatment of skin to prepare it for the topical administration of a bacteria containing formulation comprising at least one non-pathogenic *C. acnes* strain.

11. The composition according to claim 10, wherein the at least one non-pathogenic *C. acnes* strain is selected from the group of the *C. acnes* strains C3 and K8.

12. The composition according to claim 11, wherein the at least one non-pathogenic *C. acnes* strain C3 and/or K8 is combined with the *C. acnes* strains A5 and/or F4.

13. The composition according to claims 11 or 12, wherein the *C. acnes* strains C3 and K8 are used in approximately equal shares.

14. The composition according to claim 11 to 13, wherein the strains are incorporated in the form of a lyophilizate thereof.

15. A method for killing and/or reducing or inhibiting the growth of *C. acnes,* said method comprising contacting said *C. acnes* with a mixture of an ascorbyl phosphate, niacinamide and allantoin.
